# EUROPEAN PATENT APPLICATION

(11) **EP 3 900 718 A1**
(43) Date of publication of application: **27.10.2021**
(21) Application number: 19900986.1
(22) Date of filing: 20.12.2019
(51) Int. Cl.: A61K 31/198, A61P 21/00

(54) **AGENT FOR IMPROVING MUSCLE QUALITY**

(30) Priority: 21.12.2018 JP 2018240020
(71) Applicant: AJINOMOTO CO., INC., Chuo-ku Tokyo 104-8315 (JP)
(72) Inventor: MIURA, Kyoko, Kawasaki-shi, Kanagawa 210-8681 (JP); NAKAZAWA, Mai, Kawasaki-shi, Kanagawa 210-8681 (JP); KITAHARA, Yoshiro, Kawasaki-shi, Kanagawa 210-8681 (JP)
(74) Representative: Hoffmann Eitle
(86) International application number: PCT/JP2019/050188
(87) International publication number: WO 2020/130146

(57) **Abstract**

The present invention relates to a muscle quality-improving agent containing one or more selected from the group consisting of isoleucine, glycine, and cystine. According to the present invention, a muscle quality-improving agent that can prevent a decline in muscle quality, improve muscle quality even when exercise is limited, and can effectively enhance the effect of exercise even when the exercise is of a level free of undue efforts can be provided.

## Description

### [Technical Field]

The present invention relates to muscle quality-improving agents that can enhance the effect of exercise and improve muscle quality.

### [Background Art]

In recent years, with the aging of the population, health disorders accompanied by a decline in physical function such as metabolic syndrome, locomotive syndrome, and frailty (a state between a healthy state and a state requiring nursing care) have become a social problem.

To deal with the situation, various efforts have conventionally been made mainly to increase skeletal muscle mass. For example, it has been reported that ingestion of essential amino acids including a high content of leucine is effective for muscle loss (sarcopenia) in old age (non-patent document 1).

However, it has become clear that increasing skeletal muscle mass alone is not sufficient for restoring muscle strength.

In addition, it has become clear that a decline in the quality of muscle, that is, muscle quality, which was previously regarded as a change due to aging, is also caused by an unbalanced diet and lack of exercise, and leads to a serious condition such as locomotive syndrome, frailty, and the like in the future.

Exercise is known to be the most effective means of improving or preventing such decline in muscle quality. However, even if one desires to do exercise, it is often not possible to sufficiently perform exercise for various reasons such as decline in physical function due to aging, restriction on exercise due to illness, injury, etc., and the like.

Therefore, the development of a muscle quality-improving agent is desired that can prevent a decline in muscle quality, improve muscle quality, and effectively enhance the effect of exercise even when the exercise is one that can be performed without undue efforts.

### [Document List]

### [Non-patent documents]

non-patent document 1: Kim H.K. et al.; J. Am. Geriatr. Soc. 60 (1) 16-23 (2012)

### [Summary of Invention]

### [Technical Problem]

Therefore, the present invention aims to provide muscle quality-improving agents that can prevent a decline in muscle quality and improve muscle quality even when exercise is limited, and further, can effectively enhance the effect of exercise even when the exercise is of a level free of undue efforts.

### [Solution to Problem]

The present inventors have conducted intensive studies in an attempt to solve the aforementioned problems and found that one or more selected from the group consisting of isoleucine, glycine, and cystine have an effect of preventing a decline in muscle quality, or improving muscle quality, which resulted in the completion of the present invention.

Accordingly, the present invention relates to the following.
[1] A muscle quality-improving agent, comprising one or more selected from the group consisting of isoleucine, glycine, and cystine.
[2] The agent of [1], comprising isoleucine, glycine, and cystine.
[3] The agent of [2], wherein a content ratio of isoleucine, glycine, and cystine (ratio of contents converted to free form) (isoleucine:glycine:cystine) is 0.5:0.3:1 - 2:2:1 in weight ratio.
[4] The agent of [2], wherein a content ratio of isoleucine, glycine, and cystine (ratio of contents converted to free form) (isoleucine:glycine:cystine) is 0.1:0.1:1 - 10:10:1 in weight ratio.
[5] The agent of any of [1] to [3], wherein the one or more selected from the group consisting of isoleucine, glycine, and cystine are contained in a total amount of 0.001 mM - 10 mM.
[6] The agent of [4], wherein the one or more selected from the group consisting of isoleucine, glycine, and cystine are contained in a total amount of 0.001 mM - 10 mM.
[7] A medicament for improving muscle quality, comprising a muscle quality-improving agent of any of [1] to [3], and [5].
[8] A food for improving muscle quality, comprising a muscle quality-improving agent of any of [1] to [3], and [5].
[9] A medicament for improving muscle quality, comprising a muscle quality-improving agent of [4] or [6].
[10] A food for improving muscle quality, comprising a muscle quality-improving agent of [4] or [6].

### [Advantageous Effects of Invention]

The muscle quality-improving agent of the present invention can prevent a decline in muscle quality due to various reasons such as aging and the like and improve muscle quality even when exercise is limited, and further, can effectively enhance the effect of exercise even when the exercise is of a level free of undue efforts.

Therefore, the muscle quality-improving agent of the present invention is useful for preventing a decline in muscle quality and improving muscle quality even in those having difficulty in performing the exercise conventionally considered necessary for improving muscle quality, which is caused by a decline in physical function due to aging, restriction of exercise due to illness, injury, etc., and the like.

### [Brief Description of Drawings]

Fig. 1 shows an outline of evaluation of muscle insulin signal performed in Experimental Example 1.
Fig. 2 shows the effects of each of isoleucine, glycine, and cystine on muscle insulin signal in Experimental Example 1.
Fig. 3 shows the effects of a mixture of isoleucine, glycine, and cystine on muscle mitochondrial function in Experimental Example 2.
Fig. 4 shows the effects of a mixture of isoleucine, glycine, and cystine on muscle mitochondrial function under mild oxidative stress stimulation in Experimental Example 2.
Fig. 5 shows the experiment schedule of Experimental Example 3.
Fig. 6 shows the measurement of walking interval (vertical width and horizontal width) in the measurement of walking function in Experimental Example 3.
Fig. 7 shows the effects of exercise and oral administration of a mixture of isoleucine, glycine, and cystine on muscle mass in Experimental Example 3.
Fig. 8 shows the effects of exercise and oral administration of a mixture of isoleucine, glycine, and cystine on walking function in Experimental Example 3.
Fig. 9 shows the experiment schedule of Experimental Example 4.
Fig. 10 shows the effects of exercise and oral administration of a mixture of isoleucine, glycine, and cystine on muscle strength in Experimental Example 4, as compared with the effects of exercise and oral administration of a mixture of essential amino acids having a high content of leucine.

### [Description of Embodiments]

The present invention provides a muscle quality-improving agent.

The muscle quality-improving agent of the present invention (hereinafter to be also referred to as the "agent of the present invention" in the present specification) contains one or more selected from the group consisting of isoleucine, glycine, and cystine.

The "muscle quality" here refers to the quality of the muscle, or the condition of the muscle, as described above. Muscle is an aggregate of muscle fibers and tissues surrounding the muscle fibers, such as water, fat, connective tissue, and the like. In a muscle evaluated as having good muscle quality, the muscle fibers are dense and tissue surrounding the muscle fibers is less. When the number of muscle fibers decreases, or the muscle fibers become thinner, and the proportion of tissue other than muscle fibers increases, the muscle quality is evaluated to have decreased. In addition thereto, other functions of muscle such as muscle mitochondrial function, muscle insulin sensitivity, muscle inflammation, lipid metabolism, protein synthesis, and the like are also elements of muscle quality. For example, along with deterioration of muscle, lipid metabolism function decreases, fat accumulates easily, and muscle mitochondrial function and muscle protein synthesis function decrease. The decrease in these functions also leads to the evaluation of decline in muscle quality.

Therefore, "prevention of decline in muscle quality" refers to preventing muscle fibers from decreasing, muscle fibers from becoming thinner, and fat and connective tissue around muscle fibers from increasing, maintaining muscle mitochondrial function, maintaining muscle insulin sensitivity, and preventing muscle inflammation and decline in muscle lipid metabolism function and protein synthesis function. "Improvement of muscle quality" refers to increasing muscle fibers or making muscle fibers thick and dense, decreasing fat and connective tissue around muscle fibers, improving muscle inflammation and insulin sensitivity, and improving the intrinsic metabolic functions of muscles such as muscle mitochondrial function, lipid metabolism function, protein synthesis function, and the like to place muscles in good condition.

The "muscle quality-improving agent" in the present invention refers to one having a function to prevent the above-mentioned decline in muscle quality, or one having a function to improve muscle quality, or one having the both functions.

As a result of the prevention of a decline in the muscle quality or improvement of the muscle quality by the agent of the present invention, the muscle strength is improved, and improvement of walking speed and walking function, improvement and enhancement of functions in daily activities, such as improvement of standing-up and sitting-down, improvement of ascending and descending of stairs, maintaining a standing posture, and the like, improvement of exercise performance, such as improvement of exercise endurance, improvement of instantaneous force, and the like, and the like are expected.

The cystine contained as an active ingredient in the agent of the present invention has a structure in which two cysteine molecules are linked via a disulfide bond (S-S) generated by oxidation of a sulfhydryl group (-SH).

As the isoleucine and cystine, any of L-form, D-form and DL-form may be used. The L-form and DL-form are preferably used, and L-form is more preferably used.

In addition, "isoleucine", "glycine" and "cystine" can be used not only in a free form but also a salt form. The terms "isoleucine", "glycine" and "cystine" in the present specification are each a concept encompassing even a salt. The salt form is not particularly limited as long as it is a pharmacologically acceptable salt, and acid addition salt, salt with base and the like can be mentioned.

Specifically, salts with inorganic bases, salts with organic bases, salts with inorganic acids, salts with organic acids, salts with amino acid and the like can be mentioned.

Examples of the salts with inorganic bases include salts with alkali metals such as lithium, sodium, potassium and the like, salts with alkaline earth metals such as magnesium, calcium and the like, ammonium salt and the like.

Examples of the salts with organic bases include salts with alkanolamine such as monoethanolamine, diethanolamine, triethanolamine and the like, salts with heterocyclic amine such as morpholine, piperidine and the like, and the like.

Examples of the salts with inorganic acids include salts with hydrohalic acid (hydrochloric acid, hydrobromic acid, hydroiodic acid, etc.), sulfuric acid, nitric acid, phosphoric acid and the like, and the like.

Examples of the salts with organic acids include salts with monocarboxylic acid such as formic acid, acetic acid, propanoic acid and the like; salts with saturated dicarboxylic acid such as oxalic acid, malonic acid, malic acid, succinic acid and the like; salts with unsaturated dicarboxylic acid such as maleic acid, fumaric acid and the like; salts with tricarboxylic acid such as citric acid and the like; salts with keto acid such as α-ketoglutaric acid and the like, and the like.

Examples of the salts with amino acid include salts with aliphatic amino acid such as alanine and the like; salts with aromatic amino acid such as tyrosine and the like; salts with basic amino acid such as arginine and the like; salts with acidic amino acid such as aspartic acid, glutamic acid and the like; salts with amino acid forming lactam such as pyroglutamic acid and the like; and the like.

The above-mentioned salts may each be a hydrate (hydrate salt), and examples of the hydrate include 1 hydrate - 6 hydrate and the like.

In the present invention, one of "isoleucine", "glycine" and "cystine" in the above-mentioned free form or a salt form may be used singly, or two or more thereof may be used in combination.

Each of "isoleucine", "glycine" and "cystine" in a free form and hydrochloride thereof and the like are preferably used for the purpose of the present invention.

In the present invention, "isoleucine", "glycine" and "cystine" in a free form or in the form of a salt to be used may be extracted from animals, plants or the like, which are naturally present, and purified, or obtained by a chemical synthesis method, a fermentation method, an enzyme method or a gene recombinant method. Commercially available products provided by each company may also be utilized.

The agent of the present invention can contain one or more selected from the group consisting of isoleucine, glycine, and cystine.

From the aspect of muscle quality-improving effect, it is preferable to contain two selected from the group consisting of isoleucine, glycine, and cystine in combination, and it is more preferable to use all of isoleucine, glycine, and cystine as a mixture.

When all of isoleucine, glycine, and cystine are contained, they are preferably contained such that the content ratio of these (ratio of contents converted to free form) (isoleucine:glycine:cystine) is 0.5:0.3:1 - 2:2:1 in a weight ratio.

In another embodiment of the present invention, when all of isoleucine, glycine, and cystine are contained, they are preferably contained such that the content ratio of these (ratio of contents converted to free form) (isoleucine:glycine:cystine) is 0.1:0.1:1 - 10:10:1 in a weight ratio.

From the aspect of muscle quality-improving effect, moreover, the agent of the present invention is preferably composed of isoleucine, glycine, and cystine alone.

When the agent of the present invention is in a liquid form, the content of one or more selected from the group consisting of isoleucine, glycine, and cystine is generally 0.001 mM - 10 mM, preferably 0.01 mM - 5 mM, more preferably 0.1 mM - 3 mM, in a total amount of these.

The agent of the present invention can also contain other nutritional supplements and anti-fatigue agent and the like. As the nutrition component and the like, carbohydrate preparation such as glucose, dextran and the like, fat emulsion such as purified soybean oil, purified egg-yolk lecithin and the like, protein preparation such as casein, whey protein and the like, caffeine, vitamins, minerals, polyphenols and the like can be specifically mentioned.

The agent of the present invention can also contain an amino acid other than isoleucine, glycine or cystine.

The agent of the present invention can have a dosage form of an oral preparation such as tablet, coating tablet, chewable tablet, pill, (micro)capsule, granule, fine granule, powder, elixir, lemonade, syrup, suspension, emulsion, oral jelly or the like, an injectable preparation, for example, an injection such as solution, suspension, emulsion or the like, a solid injection to be used by dissolving or suspending when in use, a transfusion, a sustainable injection or the like, and the like.

The agent of the present invention in the above-mentioned dosage form can be prepared by a formulating means well known in the field of preparations, for example, the methods described in the Japanese Pharmacopoeia, seventeenth Edition, General Rules for preparation, [3] Monographs for Preparations.

In this case, various pharmacologically acceptable additives for preparations can be blended as necessary. The additive can be appropriately selected according to the dosage form of the agent of the present invention. For example, excipient, binder, disintegrant, lubricant, coating agent, base, solvent, diluent, solubilizing agent, solubilizer, emulsifier, dispersing agent, suspending agent, stabilizer, thickener, soothing agent, isotonicity agent, pH adjuster, antioxidant, antiseptic, preservative, corrigent, flavoring agent, sweetening agent, flavor, colorant and the like can be mentioned.

Specifically, examples of the excipient include magnesium carbonate, titanium dioxide, saccharides (lactose, etc.), sugar alcohol (mannitol, etc.), casein and the like.

Examples of the binder include gelatin, starch, cellulose and a derivative thereof and the like.

Examples of the disintegrant include crospovidone, crystalline cellulose and the like.

Examples of the lubricant include talc, magnesium stearate and the like.

Examples of the coating agent include methylmethacrylate. butylmethacrylate_{•}dimethylaminoethylmethacrylate copolymer, ethylacrylate_{•}methylmethacrylate_{•} trimethylammmonioethylmethacrylate chloride copolymer and the like.

Examples of the base include animal oil, vegetable oil, hydrocarbon oil (liquid paraffin, etc.), polyethylene glycol and the like.

Examples of the solvent include purified water, water for injection, monovalent alcohol (ethanol, etc.), polyhydric alcohol (glycerol, etc.) and the like.

Examples of the emulsifier or dispersing agent include sorbitan fatty acid ester, glycerine fatty acid ester, polyoxyethylene sorbitan fatty acid ester, sucrose fatty acid ester and the like.

Examples of the stabilizer include adipic acid, β-cyclodextrin and the like.

Examples of the thickener include water-soluble polymer (sodium polyacrylate, carboxyvinyl polymer, etc.), polysaccharides (sodium alginate, xanthan gum, tragacanth, etc.) and the like.

Examples of the soothing agent include ethyl aminobenzoate, chlorobutanol, propylene glycol, benzyl alcohol and the like.

Examples of the isotonicity agent include potassium chloride, sodium chloride, sorbitol, physiological saline and the like.

Examples of the pH adjuster include hydrochloric acid, sulfuric acid, acetic acid, citric acid, lactic acid, sodium hydroxide, potassium hydroxide and the like.

Examples of the antioxidant include dibutylhydroxytoluene (BHT), butylhydroxyanisole (BHA), α-tocopherol, erythorbic acid and the like.

Examples of the antiseptic or preservative include paraben (methylparaben, etc.), benzyl alcohol, sodium dehydroacetate, sorbic acid and the like.

Examples of the corrigent or flavoring agent include ascorbic acid, erythritol, sodium L-glutamate and the like.

Examples of the sweetening agent include aspartame, licorice extract, saccharin and the like.

Examples of the flavor include l-menthol, d-camphor, cineol and the like.

Examples of the colorant include tar pigment (red No. 2, blue No. 1, yellow No. 4, etc.), inorganic pigment (red iron oxide, yellow iron oxide, black iron oxide, etc.), natural dye (annatto dye, turmeric dye, β-carotene, etc.) and the like.

The ingestion amount or dose of the agent of the present invention is appropriately determined according to the condition of muscle quality or degree of decline in muscle quality, gender, age, and body weight of the subject to whom the agent of the present invention is applied (hereinafter to be referred to as the "application target" in the present specification), dosage form of the agent of the present invention, administration method and the like. When the application target is a human adult, the total amount of one or more selected from the group consisting of isoleucine, glycine, and cystine (when contained in a salt form, an amount converted to a free form) is generally 1 mg/kg body weight - 2 g/kg body weight, preferably 5 mg/kg body weight - 1 g/kg body weight, more preferably 10 mg/kg body weight - 0.5 g/kg body weight, per day.

The above-mentioned amount of ingestion or dose can be taken once or in two or more portions (e.g., 2 to 5 portions) per day.

The timing of ingestion or administration of the agent of the present invention is not particularly limited, and may be ingested or administered before or after meal, or together with meal. When exercise is performed, it may be ingested or administered at any timing of before start of the exercise, during the exercise, after completion of the exercise or the like.

While the number of ingestion or administration of the agent of the present invention is not particularly limited, it is at least once (once or twice or more) when the muscle quality needs to be improved.

When the number of ingestions or administrations of the agent of the present invention is 2 or more, while the ingestion or administration period (period from the first ingestion or administration to the last ingestion or administration) of the agent of the present invention is not particularly limited, it is generally 6 hr to 4 weeks. To exhibit the effect more, it is preferably 1 day to 2 weeks, more preferably 3 days to 1 week.

Since isoleucine, glycine, and cystine contained in the agent of the present invention are amino acids with rich food experience and high safety, the agent of the present invention can be ingested or administered continuously. In particular, it is preferably ingested or administered for a long period of time (e.g., 2 weeks or more) to prevent a decline in muscle quality.

The agent of the present invention can be formulated as a unit package form. In the present specification, the "unit package form" means a form of one or more units with a particular amount (e.g., intake per one time, etc.) as one unit is/are packed in one container or package. For example, a unit package form with intake per one time as one unit is referred to as "unit package form for intake per one time". A container or package used for the unit package form can be appropriately selected according to the form and the like of the agent of the present invention. For example, paper container or bag, plastic container or bag, pouch, aluminum can, steel can, glass bottle, pet bottle, PTP (press through pack) package sheet and the like can be mentioned.

The application target of the agent of the present invention includes mammals (human, mouse, rat, hamster, rabbit, cat, dog, bovine, horse, donkey, swine, sheep, monkey, etc.) and birds (chicken, etc.) and the like. When the agent of the present invention is applied to an application target animal other than human (hereinafter to be also simply referred to as "target animal"), the ingestion amount or dose of the agent of the present invention can be appropriately set according to the kind, sex, body weight and the like of the target animal.

The agent of the present invention can prevent a decline in muscle quality due to various reasons such as aging and the like and improve muscle quality even when exercise is limited, and further, can effectively enhance the effect of exercise even when the exercise is of a level free of undue efforts.

Therefore, the agent of the present invention is preferably used for preventing a decline in muscle quality or improving muscle quality in those having difficulty in performing the exercise conventionally considered necessary for improving muscle quality, such as elderly people experiencing a decline in physical function due to aging, those under restriction of exercise due to illness, injury, etc., and the like.

In addition, the agent of the present invention is also preferably used for those who are obese and desire to control their weight through exercise, those who are slightly underweight and desire to improve their physical strength through exercise, exercise enthusiasts who wish to improve their exercise performance, and the like.

The agent of the present invention can be used as it is or added with the above-mentioned additives such as excipient, solvent, diluent and the like to give a medicament for improving muscle quality (hereinafter to be also referred to as "the medicament of the present invention" in the present specification).

The total content of one or more selected from the group consisting of isoleucine, glycine, and cystine in the medicament of the present invention (when contained in a salt form, an amount converted to a free form) is generally 0.02 wt% - 100 wt%, preferably 0.1 wt% - 100 wt%, more preferably 0.2 wt% - 100 wt%.

The dose of the medicament of the present invention can be appropriately determined according to the condition and degree of decline in muscle quality of patients to whom the medicament of the present invention is administered, and the age, gender, body weight and the like of the patients. It can be determined such that the dose of one or more selected from the group consisting of isoleucine, glycine, and cystine is the above-mentioned daily dose.

The medicament of the present invention can be produced by a means of formulation well known in the field of pharmaceutical preparation, such as the method described in the Japanese Pharmacopoeia, seventeenth Edition, General Rules for Preparation, [3] Monographs for Preparations, and the like.

The medicament of the present invention can be suitably administered to elderly people, patients, persons in need of nursing care and the like who show a decline in muscle quality or are at a risk of a decline in muscle quality.

Furthermore, the agent of the present invention can be used by adding to various foods. The food to which the agent of the present invention is added is not particularly limited, and may be any as long as it is a food, dessert, or the like in the form generally served for meals. For example, the agent of the present invention is added to drinks, and a suitable flavor is added when desired, whereby a drink (e.g., beverage etc.) can be provided. More specifically, the agent of the present invention can be added to, for example, juice, milk, confectionery, jelly, yogurt, candy and the like.

The agent of the present invention may be added to a food in an amount to be ingested per day such that the ingestion amount of one or more selected from the group consisting of isoleucine, glycine, and cystine is the above-mentioned daily ingestion amount.

The present invention also provides a food for improving muscle quality containing the agent of the present invention (hereinafter to be also referred to as "the food of the present invention").

The food of the present invention contains the agent of the present invention and, where necessary, food additives such as production agent, thickening stabilizer, gum base, emulsifier, preservation, antioxidant, gloss agent, pH adjuster, sweetener, bitter taste, acidulant, colorant, flavor and the like. Alternatively, the food composition of the present invention can be provided in various forms containing the agent of the present invention and food or food starting materials, for example, drinks such as juice, beverage water, teas and the like; milk products such as lactobacillus drinks, fermented milk, butter, cheese, yogurt, processing milk, defatted milk and the like; meat products such as ham, sausage, hamburg steak and the like; fish meat paste products such as boiled fish paste, tube-like fish sausage, *satsuma-age* and the like; egg products such as rolled Japanese-style omelette, egg tofu and the like; confectioneries such as cookie, jelly, chewing gum, candy, snack confectionery, frozen dessert and the like; bread; noodles; pickles; smoked product; dried fish; food boiled down in soy; salt-preserved product; soups; seasonings, and may be provided as bottled food, canned food, retort pouch food. In addition, forms such as powder, granule, sheet, capsule, tablet, jelly and the like can be provided.

The food of the present invention can be preferably ingested by elderly people, person in need of nursing care, patients and the like who are at a risk of a decline in muscle quality or requesting improvement of muscle quality.

In addition, the food of the present invention can be preferably ingested widely by, besides elderly people, middle-aged people who desire to prevent a decline in muscle quality or improve muscle quality, those who are obese and desire to control their weight through exercise, those who are slightly underweight and desire to improve their physical strength through exercise, those who do not require nursing care but are under restriction in performing exercise due to illness, injury, etc., and those who wish to improve their muscle quality, such as exercise enthusiasts who wish to improve their exercise performance, and the like.

Therefore, the food of the present invention can also be provided as food with health claims such as food for specified health uses, food with nutrient function, food with functional claims and the like for preventing a decline in muscle quality or improving muscle quality, special purpose foods such as food for sick people, food for the elderly and the like, health supplement and the like.

Furthermore, the agent of the present invention can be used by adding to a high density liquid diet or food supplement.

The "high density liquid diet" is a comprehensive nutritional food (liquid diet) adjusted to a nutritional calorific value of about 1 kcal/mL, which is designed based on the daily nutritional requirement and with sufficient consideration of the qualitative composition of each nutrient so that remarkable excessive or insignificant nutrients will not occur even when only this is ingested for a long period of time.

The "food supplement" in the present invention refers to one ingested to aid nutrition other than one ingested as a food, and also includes nutritional supplement, supplement and the like. When the agent of the present invention is added to a food supplement, it can be prepared in a form such as tablet, capsule, powder, granule, suspension, chewable, syrup and the like by adding other nutrition components and additives when desired.

The above-mentioned food of the present invention can be processed and produced by adding food additive as necessary to the agent of the present invention or adding the agent of the present invention to a food or food starting materials, and applying a general food production method.

The content of one or more selected from the group consisting of isoleucine, glycine, and cystine in the food of the present invention can be appropriately determined according to the kind or form of the food, the level of the muscle quality improving effect expected by the ingestion of the food and the like. The total content of one or more selected from the group consisting of isoleucine, glycine, and cystine (when contained in a salt form, an amount converted to a free form) is generally about 0.02 wt% - 100 wt%, preferably about 0.1 wt% - 100 wt%, more preferably about 0.2 wt% - 100 wt%, of the total weight of the food.

The daily ingestion amount of the food of the present invention can be set as an amount that can achieve ingestion of the aforementioned daily ingestion amount of one or more selected from the group consisting of isoleucine, glycine, and cystine in the agent of the present invention.

The present invention also provides a commercial package containing the agent of the present invention and a written matter stating that the agent of the present invention can or should be used for improving muscle quality.

Furthermore, the present invention also provides a method for improving muscle quality of a target animal in need of improvement of the muscle quality (hereinafter to be also referred to as "the method of the present invention" in the present specification).

The method of the present invention comprises ingestion or administration of one or more selected from the group consisting of isoleucine, glycine, and cystine in an amount effective for improving muscle quality of a target animal in need of improvement of the muscle quality.

As the target animal of the method of the present invention, human and mammals other than human such as mouse, rat, hamster, rabbit, cat, dog, bovine, horse, donkey, swine, sheep, monkey and the like, and birds such as chicken and the like can be mentioned.

In the case of human, the method of the present invention can be widely applied to a person showing a decline in muscle quality, and a person who desires prevention of a decline in the muscle quality or improvement of the muscle quality. Particularly, it is preferably applied to patients and the like who cannot perform sufficient exercise, such as elderly people and persons in need of nursing care who are experiencing a decline in physical function, those under restriction of exercise due to illness, injury, etc., and the like.

The ingestion amount or dose of one or more selected from the group consisting of isoleucine, glycine, and cystine in the method of the present invention is appropriately determined according to the kind, age, gender, body weight, condition and degree of a decline in muscle quality of the target animal, and the like. An amount similar to the above-mentioned ingestion amount or dose of the agent of the present invention for a human or a target animal other than human can be ingested or administered at the frequency and period mentioned above.

The ingestion or administration method of one or more selected from the group consisting of isoleucine, glycine, and cystine in the method of the present invention includes oral ingestion, oral administration, enteral tube administration, administration by infusion and the like. Oral ingestion or oral administration is preferable since convenient ingestion is possible without the need to perform under the guidance and supervision of a doctor at a medical institution.

### [Example]

The present invention is explained in more detail in the following by way of Experimental Examples.

In the following Experimental Examples, "isoleucine" is L-isoleucine and "cystine" is L-cystine.

### [Experimental Example 1] Study of action of isoleucine, glycine, and cystine on decrease in muscle insulin signal

Muscle anabolic signal was monitored by insulin signal, and the respective actions of isoleucine, glycine, and cystine on decrease of insulin signal were evaluated.

The evaluation was performed using an evaluation system in which exposure of the mouse-derived muscle cell line C2C12 to saturated fatty acid results in a decrease in insulin signal, as described below.

### (1) Differentiation induction into C2C12 myotube cell

### (Myotubes)

Using mouse C3H skeletal muscle myoblast (C2C12 cell) (DS Pharma Biomedical Co., Ltd.), the evaluation was performed.

Induction of differentiation into myotube cells (Myotubes) was performed using cells with myoblast passage number of 12 - 18. Myoblasts were seeded in a 12-well (diameter=3 cm) plate at 0.3×10⁵ - 0.5×10⁵/well. After confirmation that the cell proliferation state reached 90% confluent, the medium was replaced with DMEM culture medium supplemented with 2(w/v)% horse serum (HS) and 1(w/v)% penicillin-streptomycin-glutamine (2(w/v)% HS, 1(w/v)% P/S, 1(w/v)% Gln/DMEM), and differentiation into myotube cells (Myotubes) was induced. After confirmation of differentiation into myotube cells (Myotubes), a muscle insulin signal evaluation system was constructed, and the action of each of isoleucine, glycine, and cystine was evaluated using the constructed evaluation system.

### (2) Evaluation of muscle insulin signal

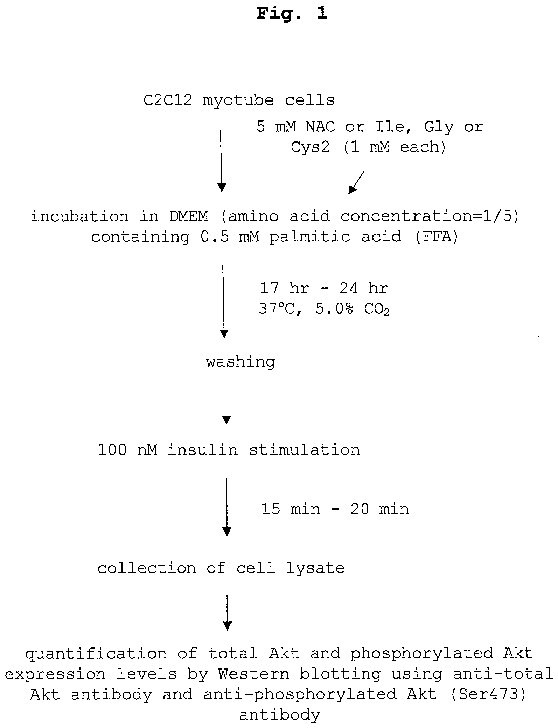

The outline of the evaluation of muscle insulin signal is shown in Fig. 1.

The myotube cells (Myotubes) were cultured overnight (17 hr - 24 hr) at 37°C in a DMEM culture medium containing 0.5 mM palmitic acid (FFA)/0.5(w/v)% albumin (BSA)/amino acid at a concentration of 1/5 in the presence of 5(v/v)% carbon dioxide.

After culturing overnight, the cells were preincubated for about 2 hr in DMEM (BSA/FFA free) with an amino acid concentration of 1/5, stimulated by incubation (15 min - 20 min) with 100 nM insulin, and the myotube cells were collected (each n=3). From the collected cells, proteins were extracted with RIPA (Radio-Immunoprecipitation Assay) buffer (Cell Signaling Technologies Inc.), adjusted to a final protein concentration of 0.5 mg/mL, and used as samples.

The expression levels of phosphorylated Akt (pAkt) (Ser473) and total Akt (total Akt) in the above-mentioned sample were quantified by the Western blotting (WB) method using anti-phosphorylated Akt (pAkt) (Ser473) antibody (# 9271S; Cell Signaling Technologies Inc.) and anti-total Akt (total Akt) antibody (#9272S; Cell Signaling Technologies Inc.). The expression level of each protein was quantified using Western blot-chemiluminescent imaging system (Fusion FX) (Vilber-Lourmat Inc.).

Each amino acid was added simultaneously with FFA, and the muscle quality-improving effect was examined with the recovery of the muscle insulin signal as an index. The final concentration of each amino acid was adjusted to 1 mM. As a positive control, an antioxidative active substance N-acetylcysteine (NAC) 5 mM was used.

### (3) Evaluation results

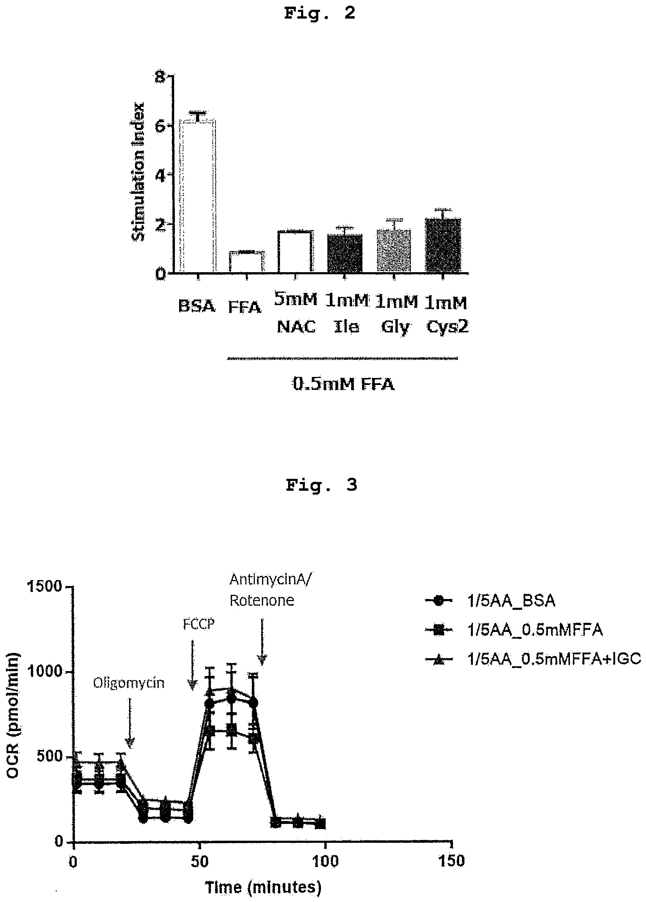

From the measurement results of the expression levels of phosphorylated Akt (Ser473) and total Akt, the ratio of phosphorylated Akt and total Akt with insulin stimulation to the ratio of phosphorylated Akt and total Akt without insulin stimulation [(pAkt/Total Akt) Insulin+]/[(pAkt/Total Akt) Insulin-] was determined and shown in Fig. 2 as the phosphorylation index (stimulation index). In Fig. 2, the phosphorylation index is shown as mean±standard error for each sample.

In Fig. 2, insulin-stimulated promotion of Akt phosphorylation was observed in myotube cells cultured with the addition of BSA. The phosphorylation of Akt by insulin stimulation was suppressed in cells cultured with the addition of 0.5 mM FFA, suggesting a decrease in muscle insulin signal.

On the other hand, it was found that isoleucine, glycine, and cystine each at a concentration of 1 mM restore the muscle insulin signal lowered by FFA. In addition, it was found that each amino acid shows an action equal to or higher than that of NAC at a concentration lower than that of NAC.

The results of this Experimental Example suggest that isoleucine, glycine, and cystine may each be useful as components that cause an increase in muscle insulin signal.

### [Experimental Example 2] Study of action of isoleucine, glycine, and cystine mixture on muscle mitochondrial function

The action of an isoleucine, glycine, and cystine mixture on a functional decline in muscle mitochondria was evaluated.

The evaluation was performed by exposing the mouse-derived muscle cell line C2C12 to saturated fatty acid and, as described below, observing a decrease in the muscle mitochondrial function, which is observed as change in the quality of skeletal muscle, with the oxygen consumption as an index. The extracellular oxygen consumption was measured using an extracellular flux analyzer XFe24 (Primetech Corporation).

### (1) Evaluation of recovery action on decreased muscle mitochondrial function

C2C12 myoblasts (1.8×10⁴ cells/well/100 uL) were seeded in an extracellular flux analyzer XFe24 measurement plate (FluxPak-XFe24 assay pack/PS:102340-100), the medium was replaced with a differentiation medium (2(w/v)% HS, 1(w/v)% P/S, 1(w/v)% GlutaMAX (trademark)-I (L-alanyl-L-glutamine) (35050-061, Gibco), 0.3 mM HEPES (4-(2-hydroxyethyl)-1-piperazineethanesulfonic acid)/DMEM culture medium) 3 - 4 days later, and the cells were cultured. After confirmation of differentiation into myotube cells (Myotubes), the cells were further cultured overnight (17 hr - 24 hr) in a DMEM culture medium containing 0.5 mM palmitic acid (FFA)/0.5(w/v)% BSA/amino acid at a concentration of 1/5, and muscle mitochondrial function was evaluated (n=3 - 4).

A mixture of isoleucine, glycine, and cystine was added simultaneously with FFA such that the final concentration of each amino acid was 0.3 mM.

The evaluation of the muscle mitochondrial function by flux analyzer XFe24 was performed by, according to the measurement method proposed by Primetech Corporation, successively adding 3.0 µM oligomycin (ATP synthase inhibitor; 75351, Sigma Ltd.), 3.0 µM carbonyl cyanide-p-trifluoromethoxyphenylhydrazone (FCCP) (uncoupling agent; C2920, Sigma Ltd.), 0.5 µM antimycin A (mitochondria complex III inhibitor; A8674, Sigma Ltd.), 0.5 µM rotenone (mitochondria complex I inhibitor; R8875, Sigma Ltd.), and measuring the oxygen consumption rate (OCR).

The evaluation results are shown in Fig. 3. In Fig. 3, the measurement results of OCR are shown as mean±standard error for each sample.

In Fig. 3, the maximum breathing capacity of muscle mitochondria which is determined from the OCR value after the addition of FCCP and the OCR value after the addition of antimycin A and rotenone decreased by exposure to 0.5 mM FFA. On the other hand, the addition of a mixture of isoleucine, glycine, and cystine was found to restore the decreased maximum breathing capacity.

From the results of the above-mentioned Experimental Example, it was suggested that the mixture of isoleucine, glycine, and cystine has an effect of recovering the decrease in the muscle mitochondrial function.

### (2) Evaluation of action on muscle mitochondrial function during oxidative stress loading

Using myotube cells (Myotubes), an evaluation system was constructed in which the maximum oxygen consumption changes by oxidative stress due to the addition of 0.06 mM and 0.2 mM hydrogen peroxide (H₂O₂).

C2C12 myoblasts (1.8×10⁴ cells/well/maintenance medium 100 µL) were seeded in an extracellular flux analyzer XFe24 measurement plate (FluxPak-XFe24 assay pack/PS:102340-100), cultured in an incubator at 37°C in the presence of 5(v/v)% carbon dioxide, the medium was replaced with a differentiation medium (2(w/v)% HS, 1(w/v)% P/S, 1(w/v)% GlutaMAX (trademark)-I (L-alanyl-L-glutamine) (35050-061, Gibco), 0.3 mM HEPES (4-(2-hydroxyethyl)-1-piperazineethanesulfonic acid)/DMEM culture medium) 3 - 4 days later, and the cells were cultured. After confirmation of differentiation into myotube cells (Myotubes), the cells were cultured for 2 hr in a DMEM culture medium having an amino acid concentration of 1/5, and thereafter further cultured for 2 hr in a DMEM culture medium with hydrogen peroxide at each concentration of 0.06 mM and 0.2 mM/amino acid concentration of 1/5, and muscle mitochondrial function was evaluated (n=3 - 4).

A mixture of isoleucine, glycine, and cystine was added 2 hr before exposure to hydrogen peroxide such that the final concentration of each amino acid was 0.3 mM.

The evaluation of the muscle mitochondrial function by flux analyzer XFe24 was performed by, according to the measurement method proposed by Primetech Corporation, successively adding 3.0 µM oligomycin (ATP synthase inhibitor; 75351, Sigma Ltd.), 3.0 µM carbonyl cyanide-p-trifluoromethoxyphenylhydrazone (FCCP) (uncoupling agent; C2920, Sigma Ltd.), 0.5 µM antimycin A (mitochondria complex III inhibitor; A8674, Sigma Ltd.), 0.5 µM rotenone (mitochondria complex I inhibitor; R8875, Sigma Ltd.), and measuring the oxygen consumption rate (OCR).

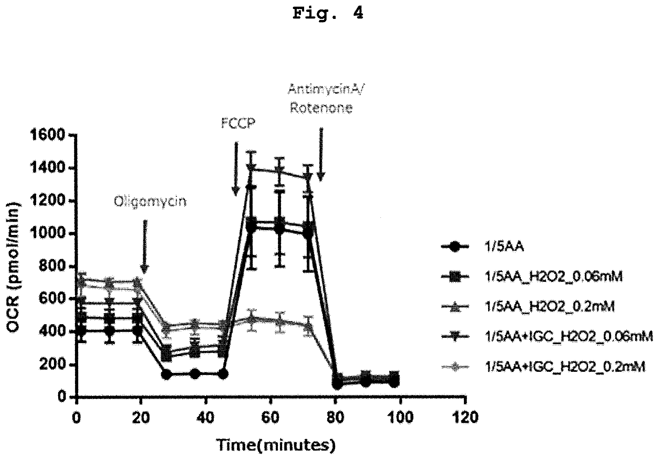

The evaluation results are shown in Fig. 4. In Fig. 4, the measurement results of OCR are shown as mean±standard error for each sample.

In Fig. 4, the maximum breathing capacity of muscle mitochondria which is determined from the OCR value after the addition of FCCP and the OCR value after the addition of antimycin A and rotenone was found to decrease by exposure to 0.2 mM hydrogen peroxide. On the other hand, such change in the maximum breathing capacity was not found by exposure to 0.06 mM hydrogen peroxide.

It was found that the maximum breathing capacity increased by exposure to 0.06 mM hydrogen peroxide when a mixture of isoleucine, glycine, and cystine was added 2 hr before the exposure to hydrogen peroxide.

Therefore, it was shown that a mixture of isoleucine, glycine, and cystine improves muscle mitochondrial function under a mild oxidative stress stimulation of a level that does not decrease the muscle mitochondrial function.

The above-mentioned mild oxidative stress stimulation is considered to reflect, for example, a situation where light exercise (jogging, walking, etc.) causes mild oxidative stress. From the results of the above-mentioned Experimental Example, isoleucine, glycine, and cystine are expected to afford an effect of improving the muscle mitochondrial function when light exercise is performed.

### [Experimental Example 3] Study of action of isoleucine, glycine, and cystine on muscle mass and muscle function (exercise function) (in vivo evaluation)

The effects of administration of isoleucine, glycine, and cystine on muscle mass and muscle function (exercise function) were evaluated as follows.

The evaluation was performed using a model animal exhibiting a decrease in muscle mass and a decline in muscle function due to intake of a high-fat diet.

6-Week-old normal Sprague-Dawley (SD) male rats (purchased from CHARLES RIVER LABORATORIES JAPAN, INC. (Kanagawa)) were acclimated and bred. At 8 weeks of age, they were divided into 4 groups (n=6/group) shown in Table 1, and supply of a normal diet (Normal) and each experimental diet of diet containing 30% by weight of fat (HF), and exercise and oral administration of a mixture of isoleucine, glycine, and cystine (IGC) were started. Isoleucine, glycine and cystine were orally administered once a day for 5 weeks at a dose of 1 g (isoleucine:glycine:cystine = 400 mg:400 mg:200 mg)/kg body weight. The amino acid mixture was dissolved in 0.5 wt% methylcellulose (MC) as a vehicle.

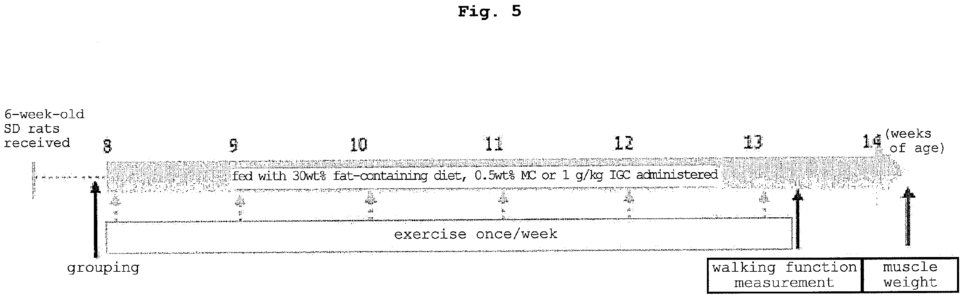

The experiment schedule is shown in Fig. 5. In the exercise group (HF/EX group and HF/EX+IGC group), exercise was performed once a week.

At the 5th week of HF supply, the exercise function (walking function) was measured, and at 6 weeks of HF supply, autopsy was performed and muscle weight was measured.

**[Table 1]**

| group | n number | feed | exercise | administered solution |
|---|---|---|---|---|
| Normal | 6 | 5 wt% fat-containing feed | no | 0.5 wt% MC |
| HF/Vehicle | 6 | 30 wt% fat-containing feed | no | 0.5 wt% MC |
| HF/EX | 6 | 30 wt% fat-containing feed | yes | 0.5 wt% MC |
| HF/EX+IGC | 6 | 30 wt% fat-containing feed | yes | 1 g/kg IGC |

The exercise was performed using the left leg of the rat by applying a local resistance exercise load. A rat under isoflurane anesthesia was placed in an ankle exercise device for small animals (Bioresearch Center K.K.), a skin stimulation electrode was attached to the anterior tibialis muscle, and contraction was caused by electrical stimulation. At the same time, the anterior tibialis muscle was pulled in the direction opposite to the contraction direction, and an extension load was applied. The exercise load setting conditions were as follows.
(i) contraction (electric) load condition: 4 mA - 5 mA, 100Hz, 1100 msec
(ii) extension load condition: angle of left leg joint was extended from 90° to 135° at rate=100 deg/sec
(iii) exercise load frequency and number of times: 10 times per 10 seconds as 1 set, and 5 sets were repeated (total 50 times), 60 sec recess between respective sets

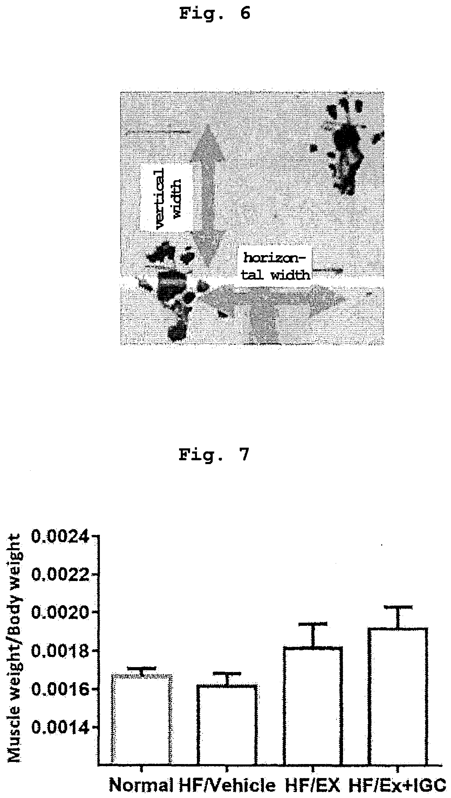

The walking function was measured by walking the rat without an anesthesia and observing the state of walking. India ink was applied to the plantar part of the both legs of the rat, and the rat was made to walk through a cylindrical tunnel with Japanese writing paper laid therein and the walking interval (horizontal width and vertical width) was measured based on the trace of the right leg and the left leg after walking, as shown in Fig. 6.

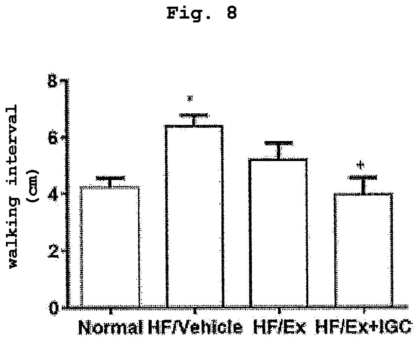

The measurement results of the muscle mass (weight of anterior tibialis muscle) are shown in Fig. 7, and the measurement results of the walking interval (horizontal width) during walking are shown in Fig. 8. The respective measurement results are shown by mean±standard error. The Tukey-Kramaer test was conducted between the Normal group and the HF/Vehicle group with respect to the respective measurement results.

In the Figure, "*" means a significant difference from the Normal group at p<0.05. In addition, "+" means a significant difference from the HF/Vehicle group at p<0.05.

In Fig. 7, the relative weight of the anterior tibialis muscle (exercise stimulation site) in the HF/Vehicle group tended to decrease as compared with the Normal group. On the other hand, in the group that performed exercise (HF/EX group), the muscle weight tended to increase compared with the HF/Vehicle group, and the muscle weight tended to further increase in the group administered with a mixture of isoleucine, glycine, and cystine in addition to exercise (HF/EX+IGC group).

In Fig. 8, the walking interval in the HF/Vehicle group was significantly broadened as compared with the Normal group, and it was thus suggested that the walking function might have decreased because the muscle strength decreased. The walking interval broadened by ingestion of a high-fat diet tended to be restored in the HF/EX group that performed exercise. It was found that in the HF/EX+IGC group administered with a mixture of isoleucine, glycine, and cystine in addition to exercise, the walking interval broadened by the ingestion of the high-fat diet significantly decreased, and it was suggested that the decreased exercise function was further improved by the administration of the mixture of isoleucine, glycine, and cystine in addition to exercise.

### [Experimental Example 4] Study of action of isoleucine, glycine, and cystine on muscle strength (in vivo evaluation)

The effects of administration of isoleucine, glycine, and cystine on muscle strength were evaluated as follows.

The evaluation was performed as in Experimental Example 3 by using a model animal exhibiting a decrease in muscle strength due to intake of a high-fat diet.

6-Week-old normal Sprague-Dawley (SD) male rats (purchased from CHARLES RIVER LABORATORIES JAPAN, INC. (Kanagawa)) were acclimated and bred. At 9 weeks of age, they were divided into 3 groups (n=6/group) shown in Table 2, and supply of an experimental diet of diet containing 30% by weight of fat (HF), and exercise and oral administration of a mixture of isoleucine, glycine, and cystine (IGC) were started. Isoleucine, glycine and cystine were orally administered once a day for 6 weeks at a dose of 1 g (isoleucine:glycine:cystine = 300 mg:170 mg:540 mg) (molar ratio=1:1:1))/kg body weight. In the exercise and high leucine-containing essential amino acid mixture administration group (HF/EX+LEAA group), the essential amino acid mixture shown in Table 3 was orally administered once a day for 6 weeks at a dose of 1 g/kg in addition to the exercise. The amino acid mixture was dissolved in 0.5 wt% methylcellulose (MC) as a vehicle.

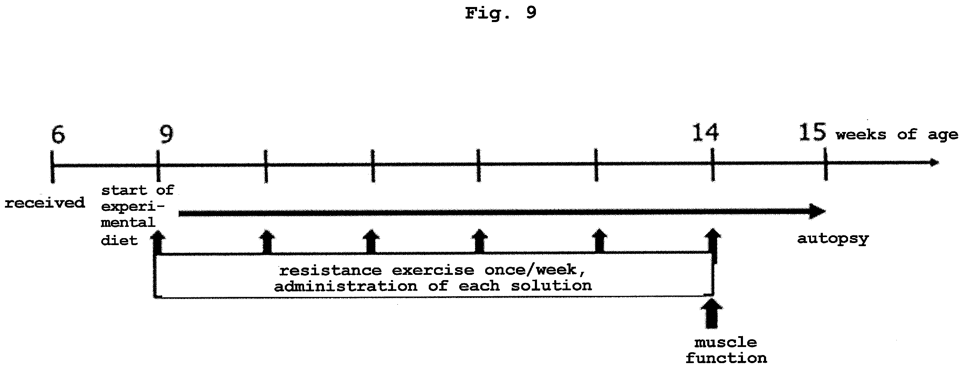

The experiment schedule is shown in Fig. 9. As the exercise, the resistance exercise was performed once a week as in Experimental Example 3.

At the 5th week of HF supply, the muscle strength was measured.

**[Table 2]**

| group | N number | feed | exercise | administered solution |
|---|---|---|---|---|
| HF/EX | 6 | 30 wt% fat-containing feed | yes | 0.5 wt% MC |
| HF/EX+IGC | 6 | 30 wt% fat-containing feed | yes | 1 g/kg IGC |
| HF/EX+LEAA | 6 | 30 wt% fat-containing feed | yes | 1 g/kg LEAA |

**[Table 3]**

| amino acid | content (wt%) |
|---|---|
| L-leucine | 40.0 |
| L-isoleucine | 10.7 |
| L-valine | 11.0 |
| L-threonine | 9.3 |
| L-lysine hydrochloride | 16.7 |
| L-methionine | 3.3 |
| L-histidine hydrochloride 1 hydrate | 1.7 |
| L-phenylalanine | 6.7 |
| L-tryptophan | 0.7 |

The muscle strength was measured using an ankle exercise device for small animals (Bioresearch center K.K.). The angles of the left leg joint and the left geniculum joint of the rat were fixed at 90°, and a skin stimulation electrode was attached to the fixed left anterior tibialis. After attaching, the muscle was stimulated with respective currents of 1.2 mA, 2.0 mA, 3.0 mA, 4.0 mA, and 4.5 mA, and the muscle contraction force for each muscle contraction stimulation was detected as muscle strength. The Tukey's test was conducted among the HF/EX group, HF/EX+IGC group and HF/EX+LEAA group with respect to the respective measurement results.

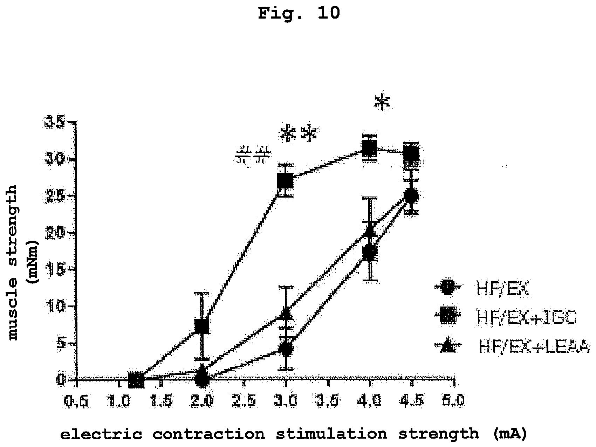

The measurement results of the muscle strength are shown in Fig. 10. The measurement results are shown by mean±standard error. In the Figure, "*" means a significant difference from the HF/EX group at p<0.05, and "**" means a significant difference from the HF/EX group at p<0.01. In addition, "##" means a significant difference from the HF/EX+LEAA group at p<0.01.

As shown in Fig. 10, in a group given a high-fat diet and an exercise (HF/EX group), an increase in the muscle strength was observed by the contraction stimulation at not less than 3.0 mA, and the maximum value (about 25 mNm) was reached by contraction stimulation at 4.5 mA.

On the other hand, in a group given a high leucine-containing essential amino acid mixture in addition to the high-fat diet ingest and exercise (HF/EX+LEAA group), while the maximum muscle strength by contraction stimulation at 4.5 mA was of the same level as the HF/EX group, the muscle strength in response to respective contraction stimulations at 3.0 mA and 4.0 mA was higher than that in the HF/EX group.

Furthermore, in a group given a high-fat diet, a mixture of isoleucine, glycine, and cystine and exercise (HF/EX+IGC group), the muscle strength increased by a contraction stimulation at 2.0 mA, a significantly high muscle strength was found by a contraction stimulation both at 3.0 mA and 4.0 mA as compared to the HF/EX group, and a remarkably high muscle strength was found by a contraction stimulation at 3.0 mA as compared to the HF/EX+LEAA group.

From the above results, it was suggested that ingestion of a mixture of isoleucine, glycine, and cystine along with exercise can cause exertion of high muscle strength even with a mild degree of contraction stimulation, and the possibility of improving the effect of exercise was suggested.

### [Industrial Applicability]

As described in detail above, the present invention can provide a muscle quality-improving agent that can prevent a decline in muscle quality due to various reasons such as aging and the like and improve muscle quality even when exercise is limited, and further, can effectively enhance the effect of exercise even when the exercise is of a level free of undue efforts.

The muscle quality-improving agent of the present invention can be effectively utilized for preventing a decline in muscle quality and improving muscle quality even in those having difficulty in performing the exercise conventionally required for improving muscle quality, such as those under restriction of exercise caused by a decline in physical function due to aging, illness, injury, etc., and the like.

In addition, the muscle quality-improving agent of the present invention can also be preferably used for those who are obese and desire to control their weight through exercise, those who are slightly underweight and desire to improve their physical strength through exercise, exercise enthusiasts who wish to improve their exercise performance, and the like.

This application is based on a patent application No. 2018-240020 filed in Japan, the contents of which are incorporated in full herein.

## Claims

1. A muscle quality-improving agent, comprising one or more selected from the group consisting of isoleucine, glycine, and cystine.

2. The agent according to claim 1, comprising isoleucine, glycine, and cystine.

3. The agent according to claim 2, wherein a content ratio of isoleucine, glycine, and cystine (ratio of contents converted to free form) (isoleucine:glycine:cystine) is 0.5:0.3:1 - 2:2:1 in weight ratio.

4. The agent according to claim 2, wherein a content ratio of isoleucine, glycine, and cystine (ratio of contents converted to free form) (isoleucine:glycine:cystine) is 0.1:0.1:1 - 10:10:1 in weight ratio.

5. The agent according to any one of claims 1 to 3, wherein the one or more selected from the group consisting of isoleucine, glycine, and cystine are contained in a total amount of 0.001 mM - 10 mM.

6. The agent according to claim 4, wherein the one or more selected from the group consisting of isoleucine, glycine, and cystine are contained in a total amount of 0.001 mM - 10 mM.

7. A medicament for improving muscle quality, comprising a muscle quality-improving agent according to any one of claims 1 to 3, and 5.

8. A food for improving muscle quality, comprising a muscle quality-improving agent according to any one of claims 1 to 3, and 5.

9. A medicament for improving muscle quality, comprising a muscle quality-improving agent according to claim 4 or 6.

10. A food for improving muscle quality, comprising a muscle quality-improving agent according to claim 4 or 6.
